(19) Europäisches Patentamt
European Patent Office
Office européen des brevets

(11) EP 0 879 887 A1

(12) **EUROPEAN PATENT APPLICATION**

(43) Date of publication:
**25.11.1998 Bulletin 1998/48**

(21) Application number: **98303809.2**

(22) Date of filing: **14.05.1998**

(51) Int Cl.[6]: **C12N 15/12**, C12N 15/85, C12N 5/10, C12Q 1/68, C07K 14/47, C07K 16/18, A61K 38/17, A61K 48/00, G01N 33/50

(84) Designated Contracting States:
**AT BE CH CY DE DK ES FI FR GB GR IE IT LI LU MC NL PT SE**
Designated Extension States:
**AL LT LV MK RO SI**

(30) Priority: **21.05.1997 US 47251 P**
**13.06.1997 US 874156**

(71) Applicant: **SMITHKLINE BEECHAM CORPORATION**
**Philadelphia Pennsylvania 19103 (US)**

(72) Inventors:
- **Hu, Erding**
  **King of Prussia, Pennsylvania 19406 (US)**
- **Zhu, Yuan**
  **King of Prussia, Pennsylvania 19406 (US)**

(74) Representative:
**Connell, Anthony Christopher et al**
**SmithKline Beecham plc**
**Corporate Intellectual Property,**
**Two New Horizons Court**
**Brentford, Middlesex TW8 9EP (GB)**

(54) **A human gene similar to a secreted murine protein SDF5 (ATG-1622)**

(57) ATG-1622 polypeptides and polynucleotides and methods for producing such polypeptides by recombinant techniques are disclosed. Also disclosed are methods for utilizing ATG-1622 polypeptides and polynucleotides in the design of protocols for the treatment of heart disease. hypertension, kidney diseases, obesity, insulin resistance, lipodystrophy, diabetes. and CNS diseases, among others, and diagnostic assays for such conditions.



Printed by Jouve, 75001 PARIS (FR)

**Annex to the description**

SEQUENCE LISTING

(1) GENERAL INFORMATION

(i) APPLICANT: SmithKline Beecham Corporation

(ii) TITLE OF THE INVENTION: A HUMAN GENE SIMILAR TO A SECRETED MURINE PROTEIN SDF5 (ATG-1622)

(iii) NUMBER OF SEQUENCES: 8

(iv) CORRESPONDENCE ADDRESS:
(A) ADDRESSEE: SmithKline Beecham, Corporate Intellectual Property
(B) STREET: Two New Horizons Court
(C) CITY: Brentford
(D) STATE: Middlesex
(E) COUNTRY: United Kingdom
(F) ZIP: TW8 9EP

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(vi) CURRENT APPLICATION DATA:
(A) APPLICATION NUMBER: 08/874,156
(B) FILING DATE: 13-JUN-1997
(C) CLASSIFICATION: UNKNOWN

(vii) PRIOR APPLICATION DATA:
(A) APPLICATION NUMBER: 60/047,251
(B) FILING DATE: 21-MAY-1997

(viii) ATTORNEY/AGENT INFORMATION:
(A) NAME: CONNELL, Anthony Christopher
(B) REGISTRATION NUMBER: 5630
(C) REFERENCE/DOCKET NUMBER: GH70028-1

(ix) TELECOMMUNICATION INFORMATION:
(A) TELEPHONE: + 44 1279 644 395
(B) TELEFAX: + 44 181 975 6294
(C) TELEX:

(2) INFORMATION FOR SEQ ID NO:1:

(i) SEQUENCE CHARACTERISTICS:
(A) LENGTH: 1984 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:1:

```
GGCTCATTCT GCTCCCCCGG GTCGGAGCCC CCCGGAGCTG CGCGCGGGCT TGCAGCGCCT     60
CGCCCGCGCT GTCCTCCCGG TGTCNNNCTT CTCCGCGCCN CAGCCGNCGG ATGCCAGCTT    120
TTCGGGGCCC CGAGTCGCAC CNAGCGAAGA GAGCGGGCCC GGGACAAGCT CGAACTCCGG    180
ACGCCTCTCC CTTCCCCGGC TCCGCTCCCT CTGCCCCCTC GGGGTCGCGC GCCCACAAAT    240
GCTGCAGGGC CCTGGCTCTC TGCTGCTGCT CTTCCTCGCC TCGCACTGCT GCCTGGGCTC    300
GGCGCGCGGG CTCTTCCTCT TTGGCCAGCC CGACTTCTCC TACAAGCGCA GCAATTGCAA    360
GCCCATCCCG GCCAACCTGC AGCTGTGCCA CGGCATCGAA TACCAGAACA TGCGGCTGCC    420
CAACCTGCTG GGCCACGAGA CCATGAAGGA GGTGCTGGAG CAGGCCGGCG CTTGGATCCC    480
GCTGGTCATG AAGCAGTGCC ACCCGGACAC CAAGAAGTTC CTGTGCTCGC TCTTCGCCCC    540
CGTCTGCCTC GATGACCTAG ACGAGACCAT CCAGCCATGC CACTCGCTCT GCGTGCAGGT    600
GAAGGACCGC TGCGCCCCGG TCATGTCCGC CTTCGGCTTC CCCTGGCCCG ACATGCTTGA    660
GTGCGACCGT TTCCCCCAGG ACAACGACCT TTGCATCCCC CTCGCTAGCA GCGACCACCT    720
CCTGCCAGCC ACCGAGGAAG CTCCAAAGGT ATGTGAAGCC TGCAAAAATA AAAATGATGA    780
TGACAACGAC ATAATGGAAA CGCTTTGTAA AAATGATTTT GCACTGAAAA TAAAAGTGAA    840
GGAGATAACC TACATCAACC GAGATACCAA AATCATCCTG GAGACCAAGA GCAAGACCAT    900
TTACAAGCTG AACGGTGTGT CCGAAAGGGA CCTGAAGAAA TCGGTGCTGT GGCTCAAAGA    960
CAGCTTGCAG TGCACCTGTG AGGAGATGAA CGACATCAAC GCGCCCTATC TGGTCATGGG   1020
ACAGAAACAG GGTGGGGAGC TGGTGATCAC CTCGGTGAAG CGGTGGCAGA AGGGGCAGAG   1080
AGAGTTCAAG CGCATCTCCC GCAGCATCCG CAAGCTGCAG TGCTAGTCCC GGCATCCTGA   1140
TGGCTCCGAC AGGCCTGCTC CAGAGCACGG CTGACCATTT CTGCTCCGGG ATCTCAGCTC   1200
CCGTTCCCCA AGCACACTCC TAGCTGCTCC AGTCTCAGCC TGGGCAGCTT CCCCCTGCCT   1260
TTTGCACGTT TGCATCCCCA GCATTTCCTG AGTTATAAGG CCACAGGAGT GGATAGCTGT   1320
TTTCACCTAA AGGAAAAGCC CACCCGAATC TTGTAGAAAT ATTCAAACTA ATAAAATCAT   1380
GAATATTTTT ATGAAGTTTA AAAATAGCTC ACTTTAAAGC TAGTTTTGAA TAGGTGCAAC   1440
```

TGTGACTTGG GTCTGGTTGG TTGTTGTTTG TTGTTTTGAG TCAGCTGATT TTCACTTCCC 1500
ACTGAGGTTG TCATAACATG CAAATTGCTT CAATTTTCTC TGTGGCCCAA ACTTGTGGGT 1560
CACAAACCCT GTTGAGATAA AGCTGGCTGT TATCTCAACA TCTTCATCAG CTCCAGACTG 1620
AGACTCAGTG TCTAAGTCTT ACAACAATTC ATCATTTTAT ACGTTCAATG GGAACTTAAA 1680
CTGTTACATG TATCACNTTC CAGCTACAAT ACTTCCATTT ATTAGAAGCA CATTAACCAT 1740
TTCTATAGCA TGATTTCTTC AAGTAAAAGG CAAAAGATAT AAATTTTATA ATTGACTTGA 1800
GTACTTTAAG CCTTGTTTAA AACATTTCTT ACTTAACTTT TGCAAATTAA ACCCATTGTA 1860
GCTTACCTGT AATATACATA GTAGTTTACC TTTAAAAGTT GTAAAAATAT TGCTTTAACC 1920
AACACTGTAA ATATTTCAGA TAAACATTAT ATTCTTGTAT ATAAACTCTA CATCCTGTTT 1980
GGGG 1984

(2) INFORMATION FOR SEQ ID NO:2:

(i) SEQUENCE CHARACTERISTICS:
(A) LENGTH: 295 amino acids
(B) TYPE: amino acid
(C) STRANDEDNESS: single
(D) TOPOLOGY: linear
(ii) MOLECULE TYPE: protein

(xi) SEQUENCE DESCRIPTION: SEQ ID NO:2:

Met Leu Gln Gly Pro Gly Ser Leu Leu Leu Leu Phe Leu Ala Ser His
1 5 10 15
Cys Cys Leu Gly Ser Ala Arg Gly Leu Phe Leu Phe Gly Gln Pro Asp
20 25 30
Phe Ser Tyr Lys Arg Ser Asn Cys Lys Pro Ile Pro Ala Asn Leu Gln
35 40 45
Leu Cys His Gly Ile Glu Tyr Gln Asn Met Arg Leu Pro Asn Leu Leu
50 55 60
Gly His Glu Thr Met Lys Glu Val Leu Glu Gln Ala Gly Ala Trp Ile
65 70 75 80
Pro Leu Val Met Lys Gln Cys His Pro Asp Thr Lys Lys Phe Leu Cys
85 90 95
Ser Leu Phe Ala Pro Val Cys Leu Asp Asp Leu Asp Glu Thr Ile Gln
100 105 110
Pro Cys His Ser Leu Cys Val Gln Val Lys Asp Arg Cys Ala Pro Val
115 120 125
Met Ser Ala Phe Gly Phe Pro Trp Pro Asp Met Leu Glu Cys Asp Arg
130 135 140
Phe Pro Gln Asp Asn Asp Leu Cys Ile Pro Leu Ala Ser Ser Asp His
145 150 155 160

```
Leu Leu Pro Ala Thr Glu Glu Ala Pro Lys Val Cys Glu Ala Cys Lys
                165                 170                 175
Asn Lys Asn Asp Asp Asp Asn Asp Ile Met Glu Thr Leu Cys Lys Asn
            180                 185                 190
Asp Phe Ala Leu Lys Ile Lys Val Lys Glu Ile Thr Tyr Ile Asn Arg
        195                 200                 205
Asp Thr Lys Ile Ile Leu Glu Thr Lys Ser Lys Thr Ile Tyr Lys Leu
    210                 215                 220
Asn Gly Val Ser Glu Arg Asp Leu Lys Lys Ser Val Leu Trp Leu Lys
225                 230                 235                 240
Asp Ser Leu Gln Cys Thr Cys Glu Glu Met Asn Asp Ile Asn Ala Pro
                245                 250                 255
Tyr Leu Val Met Gly Gln Lys Gln Gly Gly Glu Leu Val Ile Thr Ser
            260                 265                 270
Val Lys Arg Trp Gln Lys Gly Gln Arg Glu Phe Lys Arg Ile Ser Arg
        275                 280                 285
Ser Ile Arg Lys Leu Gln Cys
    290                 295
```

(2) INFORMATION FOR SEQ ID NO:3:

(i) SEQUENCE CHARACTERISTICS:

(A) LENGTH: 472 base pairs

(B) TYPE: nucleic acid

(C) STRANDEDNESS: single

(D) TOPOLOGY: linear

(ii) MOLECULE TYPE: cDNA

(xi) SEQUENCE DESCRIPTION: SEQ ID NO:3:

```
GGGCTCATTC TGCTCCCCCG GGTCGGAGCC CCCCGGAGCT GCGCGCGGGC TTGCAGCGCC     60
TCGCCCGCGC TGTCCTCCCG GTGTCCCGCT TCTCCGCGCC CCAGCCGCCG GCTGCCAGCT    120
TTTCGGGGCC CCGAGTCGCA CCCAGCGAAG AGAGCGGGCC CGGGACAAGC TCGAACTCCG    180
GCCGCCTCGC CCTTCCCCGG CTCCGCTCCC TCTGCCCCCT CGGGGTCGCG CGCCCACGAT    240
GCTGCAGGGC CCTGGCTCGC TGCTGCTGCT CTTCCTCGCC TCGCACTGCT GCCTGGGCTC    300
GGCGCGCGGG CTCTTCCTCT TTGGCCAGCC CGACTTCTCC TACAAGCGCA GAATTGCAAG    360
CCCATCCCGG CCAAACTGCA GCTGTTCCAA GGCAATCGAA TANCAGAACA TGCGGNTNGC    420
CCAAACTTGC TTGGCCANGA AGACCAATGA AAGGAAGTTN TTGGAACAAG GC            472
```

(2) INFORMATION FOR SEQ ID NO:4:

(i) SEQUENCE CHARACTERISTICS:
(A) LENGTH: 59 amino acids
(B) TYPE: amino acid
(C) STRANDEDNESS: single
(D) TOPOLOGY: linear
(ii) MOLECULE TYPE: protein

(xi) SEQUENCE DESCRIPTION: SEQ ID NO:4:

```
Met Leu Gln Gly Pro Gly Ser Xaa Xaa Xaa Xaa Xaa Xaa Ala Ser His
 1               5                  10                  15
Cys Cys Leu Gly Ser Ala Arg Gly Leu Phe Leu Phe Gly Gln Pro Asp
            20                  25                  30
Phe Ser Tyr Lys Arg Arg Ile Ala Ser Pro Ser Arg Pro Asn Cys Ser
        35                  40                  45
Cys Ser Lys Ala Ile Glu Xaa Gln Asn Met Arg
    50                  55
```

(2) INFORMATION FOR SEQ ID NO:5:

(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:5:

GAATTCATGC TGCAGGGCCC TGGCTCT 27

(2) INFORMATION FOR SEQ ID NO:6:

(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:6:

GAATTCGCAC TGCAGCTTGC GGATGCT 27

(2) INFORMATION FOR SEQ ID NO:7:

(i) SEQUENCE CHARACTERISTICS:
(A) LENGTH: 27 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:7:

GAATTCCTAG CACTGCAGCT TGCGGAT 27

(2) INFORMATION FOR SEQ ID NO:8:

(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: cDNA

(xi) SEQUENCE DESCRIPTION: SEQ ID NO:8:

GAATTCCAAA TGCTGCAGGG CCCTGGCTCT 30

## Description

## CROSS REFERENCE TO RELATED APPLICATIONS

This is a continuation-in-part application of U.S. Application Serial No. 08/874,156, filed June 13, 1997, which in turn claims the benefit of U.S. Provisional Application No. 60/047,251, filed May 21, 1997; both applications are incorporated herein by reference in their entirety.

## FIELD OF INVENTION

This invention relates to newly identified polynucleotides, polypeptides encoded by them and to the use of such polynucleotides and polypeptides, and to their production. More particularly, the polynucleotides and polypeptides of the present invention relate to secreted protein ligand for 7-TM receptor, frizzled family, hereinafter referred to as ATG-1622. The invention also relates to inhibiting or activating the action of such polynucleotides and polypeptides.

## BACKGROUND OF THE INVENTION

Protein ligands for 7-TM receptors are important molecules that regulate a variety of biological effects. Glucagon, morphin, opiate, and thrombin are all examples of these protein ligands. Glucagon. for example, elevates blood glucose levels by interacting with the glucagon receptor, a 7-TM receptor, and transducing signals to the cellular glucose regulatory machinery. Thus, identification of novel protein ligand for 7-TM receptors may reveal novel pathways to many biological processes in normal and pathological states. This indicates that the secreted protein ligand for 7-TM receptor, frizzled family has an established, proven history as therapeutic targets. Clearly there is a need for identification and characterization of further members of secreted protein ligand for 7-TM receptor, frizzled family which can play a role in preventing, ameliorating or correcting dysfunctions or diseases, including, but not limited to, heart disease, hypertension, kidney diseases, obesity, insulin resistance, lipodystropy, diabetes, and central nervous system (CNS) diseases.

## SUMMARY OF THE INVENTION

In one aspect, the invention relates to ATG-1622 polypeptides and recombinant materials and methods for their production. Another aspect of the invention relates to methods for using such ATG-1622 polypeptides and polynucleotides. Such uses include the treatment of heart disease, hypertension. kidney diseases, obesity, insulin resistance, lipodystrophy, diabetes, and CNS diseases, among others. In still another aspect, the invention relates to methods to identify agonists and antagonists using the materials provided by the invention, and treating conditions associated with ATG-1622 imbalance with the identified compounds. Yet another aspect of the invention relates to diagnostic assays for detecting diseases associated with inappropriate ATG-1622 activity or levels.

## DESCRIPTION OF THE INVENTION

### Definitions

The following definitions are provided to facilitate understanding of certain terms used frequently herein.

"ATG-1622" refers, among others, generally to a polypeptide having the amino acid sequence set forth in SEQ ID NO:2 or an allelic variant thereof.

"ATG-1622 activity or ATG-1622 polypeptide activity" or "biological activity of the ATG-1622 or ATG-1622 polypeptide" refers to the metabolic or physiologic function of said ATG-1622 including similar activities or improved activities or these activities with decreased undesirable side-effects. Also included are antigenic and immunogenic activities of said ATG-1622.

"ATG-1622 gene" refers to a polynucleotide having the nucleotide sequence set forth in SEQ ID NO:1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663:48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity," as known in the art, is a relationship between two or more polypeptide sequences or two or more polynucleotide sequences, as the case may be, as determined by comparing the sequences. In the art, "identity" also means the degree of sequence relatedness between polypeptide or polynucleotide sequences, as the case may be, as determined by the match between strings of such sequences. "Identity" can be readily calculated by known methods, including but not limited to those described in (*Computational Molecular Biology,* Lesk, A.M., ed., Oxford University Press, New York, 1988; *Biocomputing: Informatics and Genome Projects,* Smith, D.W., ed., Academic Press, New York, 1993; *Computer Analysis of Sequence Data,* Part I, Griffin, A.M., and Griffin, H.G., eds., Humana Press, New Jersey, 1994; *Sequence Analysis in Molecular Biology,* von Heinje, G., Academic Press, 1987; and *Sequence Analysis Primer,* Gribskov, M. and Devereux, J., eds., M Stockton Press, New York, 1991; and Carillo, H., and Lipman, D., SIAM *J. Applied Math., 48:* 1073 (1988). Methods to determine identity are designed to give the largest match between the sequences tested. Moreover, methods to determine identity are codified in publicly available computer programs. Computer program methods to determine identity between two sequences include, but are not limited to, the GCG program package (Devereux, J., et al., *Nucleic Acids Research 12(1):* 387 (1984)), BLASTP, BLASTN, and FASTA (Atschul, S. F. et al., *J. Molec. Biol. 215:* 403410 (1990). The BLAST X program is publicly available from NCBI and other sources (*BLAST Manual,* Altschul, S., *et al.,* NCBI NLM NIH Bethesda, MD 20894; Altschul, S., *et al., J. Mol. Biol. 215:* 403-410 (1990). The well known Smith Waterman algorithm may also be used to determine identity.

Parameters for polypeptide sequence comparison include the following:

1) Algorithm: Needleman and Wunsch, J. Mol Biol. 48: 443-453 (1970)
Comparison matrix: BLOSSUM62 from Hentikoff and Hentikoff, Proc. Natl. Acad. Sci. USA. 89:10915-10919 (1992)
Gap Penalty: 12
Gap Length Penalty: 4
A program useful with these parameters is publicly available as the "gap" program from Genetics Computer Group, Madison WI. The aforementioned parameters are the default parameters for peptide comparisons (along with no penalty for end gaps).

Parameters for polynucleotide comparison include the following:

1) Algorithm: Needleman and Wunsch, J. Mol Biol. 48: 443-453 (1970)
Comparison matrix: matches = +10, mismatch = 0
Gap Penalty: 50
Gap Length Penalty: 3
Available as: The "gap" program from Genetics Computer Group, Madison WI. These are the default parameters for nucleic acid comparisons.

A preferred meaning for "identity" for polynucleotides and polypeptides, as the case may be, are provided in (1) and (2) below.

(1) Polynucleotide embodiments further include an isolated polynucleotide comprising a polynucleotide sequence having at least a 50, 60, 70, 80, 85, 90, 95, 97 or 100% identity to the reference sequence of SEQ ID NO:1, wherein said polynucleotide sequence may be identical to the reference sequence of SEQ ID NO: 1 or may include up to a certain integer number of nucleotide alterations as compared to the reference sequence, wherein said alterations are selected from the group consisting of at least one nucleotide deletion, substitution, including transition and transversion, or insertion, and wherein said alterations may occur at the 5' or 3' terminal positions of the reference nucleotide sequence or anywhere between those terminal positions, interspersed either individually among the nucleotides in the reference sequence or in one or more contiguous groups within the reference sequence, and wherein said number of nucleotide alterations is determined by multiplying the total number of nucleotides in SEQ ID NO:1 by the integer defining the percent identity divided by 100 and then subtracting that product from said total number of nucleotides in SEQ ID NO: 1, or:

$$n_n \leq x_n - (x_n \bullet y),$$

wherein $\mathbf{n_n}$ is the number of nucleotide alterations, $\mathbf{x_n}$ is the total number of nucleotides in SEQ ID NO:1, $\mathbf{y}$ is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $\mathbf{x_n}$ and $\mathbf{y}$ is rounded down to the nearest integer prior to subtracting it from $\mathbf{x_n}$. Alterations of a polynucleotide sequence encoding the polypeptide of SEQ ID NO:2 may create nonsense, missense or frameshift mutations in this coding sequence and thereby alter the polypeptide encoded by the polynucleotide following such alterations.

By way of example, a polynucleotide sequence of the present invention may be identical to the reference sequence of SEQ ID NO:2, that is it may be 100% identical, or it may include up to a certain integer number of amino acid alterations as compared to the reference sequence such that the percent identity is less than 100% identity. Such alterations are selected from the group consisting of at least one nucleic acid deletion, substitution, including transition and transversion, or insertion, and wherein said alterations may occur at the 5' or 3' terminal positions of the reference polynucleotide sequence or anywhere between those terminal positions, interspersed either individually among the nucleic acids in the reference sequence or in one or more contiguous groups within the reference sequence. The number of nucleic acid alterations for a given percent identity is determined by multiplying the total number of amino acids in SEQ ID NO:2 by the integer defining the percent identity divided by 100 and then subtracting that product from said total number of amino acids in SEQ ID NO:2, or:

$$n_n \leq x_n - (x_n \bullet y),$$

wherein $\mathbf{n_n}$ is the number of amino acid alterations, $\mathbf{x_n}$ is the total number of amino acids in SEQ ID NO:2, **y** is, for instance 0.70 for 70%, 0.80 for 80%, 0.85 for 85% etc., • is the symbol for the multiplication operator, and wherein any non-integer product of $\mathbf{x_n}$ and **y** is rounded down to the nearest integer prior to subtracting it from $\mathbf{x_n}$.

(2) Polypeptide embodiments further include an isolated polypeptide comprising a polypeptide having at least a 50,60, 70, 80, 85, 90, 95, 97 or 100% identity to a polypeptide reference sequence of SEQ ID NO:2, wherein said polypeptide sequence may be identical to the reference sequence of SEQ ID NO: 2 or may include up to a certain integer number of amino acid alterations as compared to the reference sequence, wherein said alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence, and wherein said number of amino acid alterations is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $\mathbf{n_a}$ is the number of amino acid alterations, $\mathbf{x_a}$ is the total number of amino acids in SEQ ID NO:2, **y** is 0.50 for 50%, 0.60 for 60%, 0.70 for 70%, 0.80 for 80%, 0.85 for 85%, 0.90 for 90%, 0.95 for 95%, 0.97 for 97% or 1.00 for 100%, and • is the symbol for the multiplication operator, and wherein any non-integer product of $\mathbf{x_a}$ and **y** is rounded down to the nearest integer prior to subtracting it from $\mathbf{x_a}$.

By way of example, a polypeptide sequence of the present invention may be identical to the reference sequence of SEQ ID NO:2, that is it may be 100% identical, or it may include up to a certain integer number of amino acid alterations as compared to the reference sequence such that the percent identity is less than 100% identity. Such alterations are selected from the group consisting of at least one amino acid deletion, substitution, including conservative and non-conservative substitution, or insertion, and wherein said alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in SEQ ID NO:2 by the integer defining the percent identity divided by 100 and then subtracting that product from said total number of amino acids in SEQ ID NO:2, or:

$$n_a \leq x_a - (x_a \bullet y),$$

wherein $\mathbf{n_a}$ is the number of amino acid alterations, $\mathbf{x_a}$ is the total number of amino acids in SEQ ID NO:2, **y** is, for instance 0.70 for 70%, 0.80 for 80%, 0.85 for 85% etc., and • is the symbol for the multiplication operator, and wherein any non-integer product of $\mathbf{x_a}$ and **y** is rounded down to the nearest integer prior to subtracting it from $\mathbf{x_a}$.

**Polypeptides of the Invention**

In one aspect, the present invention relates to ATG-1622 polypeptides. The ATG-1622 polypeptides include the polypeptide of SEQ ID NOS:2 and 4; as well as polypeptides comprising the amino acid sequence of SEQ ID NO: 2; and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within ATG-1622 polypeptides are polypeptides having the amino acid sequence which have at least 80% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% are highly preferred. Preferably ATG-1622 polypeptide exhibit at least one biological activity of ATG-1622.

The ATG-1622 polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the ATG-1622 polypeptides are also included in the invention. A fragment is a polypeptide having an amino acid sequence that entirely is the same as part, but not all, of the amino acid sequence of the aforementioned ATG-1622 polypeptides. As with ATG-1622 polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of ATG-1622 polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of ATG-1622 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate ATG-1622 activity, including those with a similar activity or an improved activity, or with a decreased undesirable activity. Also included are those that are antigenic or immunogenic in an animal, especially in a human.

Preferably, all of these polypeptide fragments retain the biological activity of the ATG-1622, including antigenic activity. Among the most preferred fragment is that having the amino acid sequence of SEQ ID NO: 4. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The ATG-1622 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

**Polynucleotides of the Invention**

Another aspect of the invention relates to ATG-1622 polynucleotides. ATG-1622 polynucleotides include isolated polynucleotides which encode the ATG-1622 polypeptides and fragments, and polynucleotides closely related thereto. More specifically, ATG-1622 polynucleotide of the invention include a polynucleotide comprising the nucleotide sequence set forth in SEQ ID NOS:1 encoding a ATG-1622 polypeptide of SEQ ID NO: 2, and polynucleotides having the particular sequences of SEQ ID NOS:1 and 3. ATG-1622 polynucleotides further include a polynucleotide comprising a nucleotide sequence that has at least 80% identity to a nucleotide sequence encoding the ATG-1622 polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under ATG-1622 polynucleotides are a nucleotide sequence which has sufficient identity to a nucleotide sequence contained in SEQ ID NO:1 to hybridize under conditions useable for amplification or for use as a probe or marker. The invention also provides polynucleotides which are complementary to such ATG-1622 polynucleotides.

ATG-1622 of the invention is structurally related to other proteins of the secreted protein ligand for 7-TM receptor, frizzled family, as shown by the results of sequencing the cDNA encoding human ATG-1622. The cDNA sequence of SEQ ID NO:1 contains an open reading frame (nucleotide number 239 to 1126) encoding a polypeptide of 295 amino acids of SEQ ID NO:2. The amino acid sequence of Table 2 (SEQ ID NO:2) has about 96% identity (using blastp) in 284 amino acid residues with mouse sFRP-2 (Rattner,A., Hsieh,J.-C., Smallwood,P.M., Gilbert,D.J., Copeland,N.G., Jenkins,N.A. and Nathans,J., A family of secreted proteins containing homology to the cysteine-rich ligand-binding domain of frizzled receptors Proc. Natl. Acad. Sci. U.S.A. 94, 2859-2863, 1997). The amino acid sequence of Table 2 (SEQ ID NO:2) also has about 95% identity (using blastp) in 284 amino acid residues with Mouse SDF-5 (Shirozu,M., Tada,H., Nakamura,T., Nelson,L.D., Martina,N., Hamada,T., Sato,T., Tashiro,K., Nakano,T. and Honjo,T., Isolation of novel genes encoding for escreted or membrane proteins using signal sequence trap , Unpublished (in Genebank,

1995). The nucleotide sequence of Table 1 (SEQ ID NO:1) has about 90% identity (using blastn) in 889 nucleotide residues with mouse SDF-5 (Shirozu,M., Tada,H., Nakamura,T., Nelson,L.D., Martina,N., Hamada,T., Sato,T., Tashiro, K., Nakano,T. and Honjo,T., Isolation of novel genes encoding for excreted or membrane proteins using signal sequence trap , Unpublished (in Genebank) (1995). The nucleotide sequence of Table 1 (SEQ ID NO:1) also has about 93% identity (using blastn) in 833 nucleotide residues with Mouse sFRP-2 (Rattner,A., Hsieh,J.-C., Smallwood,P.M., Gilbert, D.J., Copeland,N.G., Jenkins,N.A. and Nathans,J., A family of secreted proteins containing homology to the cysteine-rich ligand-binding domain of frizzled receptors Proc. Natl. Acad. Sci. U.S.A. 94, 2859-2863 1997).

**Table 1[a]**

| |
|---|
| GGCTCATTCTGCTCCCCCGGGTCGGAGCCCCCCGGAGCTGCGCGCGGGCTTGCAGCGCCTCGCCCGCGCTGT |
| CCTCCCGGTGTCNNNCTTCTCCGCGCCNCAGCCGNCGGATGCCAGCTTTTCGGGGCCCCGAGTCGCACCNAG |
| CGAAgAGAgCGGGCCCGGGACAAGCTCGAACTCCGGACGCCTCTCCCTTCCCCGGCTCCGCTCCCTCTGCCC |
| CCTCGGGGTCgCGCGCCCACAAATGCTGCAgGGCCCTGGCTCTCTGCTGCTGCTCTTCCTCGCCTCGCACTG |
| CTGCCTGgGCTCGGCGCGCGGGCTCTTCCTCTTTGGCCAGCCCGACTTCTCCTACAAGCGCAGCAATTGCAA |
| GCCCATCCCGGCCAACCTGCAGCTGTGCCACGGCATCGAATACCAGAACATGCGGCTGCCCAACCTGCTGGG |
| CCACGAGACCATGAAGGAGGTGCTGGAGCAGGCCGGCGCTTGGATCCCGCTGGTCATGAAGCAGTGCCACCC |
| GGACACCAAGAAGTTCCTGTGCTCGCTCTTCGCCCCCGTCTGCCTCGATGACCTAGAcGAgAcCATCCAGCC |
| ATGCCACTCGCTCTGCGTgCAGGTgAAGGAcCGCTGCGCCCCGGTCATGTcCGCCTTCGGCTTCCCCTGGCC |
| CgACATGCTTGAgTGCGAcCGTTTCCCCCaGGAcAACGAcCTTTGCATCCCCCTCGCTAGCAGCGAcCACCT |
| CCTGCCAGCCACCGAGGAAGCTCCAAAGGTATGTGAAGCCTGCAAAAATAAAAATGATGATGACAACGACAT |
| AATGGAAACGCTTTGTAAAAATGATTTTGCACTGAAAATAAAAGTGAAGGAGATAACCTACATCAACCGAGA |
| TACCAAAATCATCCTGGAGACCAAGAGCAAGACCATTTACAAGCTGAACGGTGTGTCCGAAAGGGACCTGAA |
| GAAATCGGTGCTGTGGCTCAAAGACAGCTTGCAGTGCACCTGTGAGGAGATGAACGACATCAACGCGCCCTA |
| TCTGGTCATGGGACAGAAACAGGGTGGGGAGCTGGTGATCACCTCGGTGAAGCGGTGGCAGAAGGGGCAGAg |
| AGAGTTCAAGCGCATCTCCCGCAGCATCCGCAAGCTGCAGTGCTAGTCCCGGCATCCTGATGGCTCCGACAG |
| GCCTGCTCCAGAgCACGGCTGACCATTTCTGCTCCGGGATCTCaGcTCCCGTTCCCCAAGCACAcTCcTAGc |
| TGcTCCAGTcTCAGCctGGGCAGCTTCCCCcTGCCTTTtGCACGTTTGCATCCCCAGCATTTCCTGAGTTAT |
| AAGGCCACAGGAGTGGATAGCTGTTTTCACCTAAAGGAAAAGCCCACCCGAATCTTGTAGAAATATTCAAAC |
| TAATAAAATCATGAATATTTTTATGAAGTTTAAAAATAGCTCACTTTAAAGCTAGTTTTGAATAGGTGCAAC |
| TGTGACTTGGGTCTGGTTGGTTGTTGTTTGTTGTTTTGAGTCAGCTGATTTTCACTTCCCACTGAGGTTGTC |
| ATAACATGCAAATTGCTTCAATTTTCTCTGTGGCCCAAACTTGTGGGTCACAAACCCTGTTGAGATAAAGCT |
| GGCTGTTATCTCAACATCTTCATCAGCTCCAGACTGAGACTCAGTGTCTAAGTCTTACAACAATTCATCATT |
| TTATACGTTcaatGGGAACTTAAAcTGTTACATGTATCACNTTCCAGcTACAATACTTCCATTtattaGAAG |
| CACATTAACCATTTCTATaGCATGATTTCTTCAAGTAAAAGGCAAAAGATATAAATTTTATAATtGActtGA |
| GTACTtTAAGCCTTGTTTAAAACATTTCTTACTTAACTTTTGCAAATTAAACCCATTGTAGCTTACCTGTAA |
| TATACATAGTAGTttACCTTTAAAAGTTGTAAAAATATTGCTttaACCAACACTGTAAATATTTCAGATAAA |
| CATTATATTCTTGTATATAAACTCtACATCCTGTTTGGGG |

[a] A nucleotide sequence of a human ATG-1622 (SEQ ID NO: 1).

**Table 2[b]**

| |
|---|
| MLQGPGSLLLLFLASHCCLGSARGLFLFGQPDFSYKRSNCKPIPANLQLCHGIEYQNMRLPNLLGHETMKEV |
| LEQAGAWIPLVMKQCHPDTKKFLCSLFAPVCLDDLDETIQPCHSLCVQVKDRCAPVMSAFGFPWPDMLECDR |
| FPQDNDLCIPLASSDHLLPATEEAPKVCEACKNKNDDDNDIMETLCKNDFALKIKVKEITYINRDTKIILET |
| KSKTIYKLNGVSERDLKKSVLWLKDSLQCTCEEMNDINAPYLVMGQKQGGELVITSVKRWQKGQREFKRISR |
| SIRKLQC. |

[b] An amino acid sequence of a human ATG-1622 (SEQ ID NO: 2).

One polynucleotide of the present invention encoding ATG-1622 may be obtained using standard cloning and screening, from a cDNA library derived from mRNA in cells of human Hodgkin's lymphoma II using the expressed sequence tag (EST) analysis (Adams, M.D., *et al. Science* (1991) 252:1651-1656; Adams, M.D. *et al., Nature,* (1992) *355*:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding ATG-1622 polypeptide of SEQ ID NO:2 may be identical to the polypeptide encoding sequence contained in Table 1 (nucleotide number 239 to 1126 of SEQ ID NO:1), or it may be a sequence, which as a result of the redundancy (degeneracy) of the genetic code, also encodes the polypeptide of SEQ ID NO:2.

When the polynucleotides of the invention are used for the recombinant production of ATG-1622 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding ATG-1622 variants comprising the amino acid sequence of ATG-1622 polypeptide of Table 2 (SEQ ID NO:2) in which several, 5-10, 1-5, 1-3, 1-2 or 1 amino acid residues are substituted, deleted or added, in any combination. Among the preferred polynucleotides of the present invention is contained in Table 3 (SEQ ID NO: 3) encoding the amino acid sequence of Table 4 (SEQ ID NO: 4).

**Table 3[c]**

| |
|---|
| GGGCTCATTCTGCTCCCCCGGGTCGGAGCCCCCCGGAGCTGCGCGCGGGCTTGCAGCGCC |
| TCGCCCGCGCTGTCCTCCCGGTGTCCCGCTTCTCCGCGCCCCAGCCGCCGGCTGCCAGCT |
| TTTCGGGGCCCCGAGTCGCACCCAGCGAAGAGAGCGGGCCCGGGACAAGCTCGAACTCCG |
| GCCGCCTCGCCCTTCCCCGGCTCCGCTCCCTCTGCCCCCTCGGGGTCGCGCGCCCACGAT |
| GCTGCAGGGCCCTGGCTCGCTGCTGCTGCTCTTCCTCGCCTCGCACTGCTGCCTGGGCTC |
| GGCGCGCGGGCTCTTCCTCTTTGGCCAGCCCGACTTCTCCTACAAGCGCAGAATTGCAAG |
| CCCATCCCGGCCAAACTGCAGCTGTTCCAAGGCAATCGAATANCAGAACATGCGGNTNGC<br>CCAAACTTGCTTGGCCANGAAGACCAATGAAAGGAAGTTNTTGGAACAAGGC |

[c] A partial nucleotide sequence of a human ATG-1622 (SEQ ID NO: 3).

**Table 4[d]**

| |
|---|
| MLQGPGSXXXXXXASHCCLGSARGLFLFGQPDFSYKRRIASPSRPNCSCSKAIEXQNMR |

[d] A partial amino acid sequence of a human ATG-1622 (SEQ ID NO: 4).

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof (including that of SEQ ID NO:3), may be used as hybridization probes for cDNA and genomic DNA, to isolate full-length cDNAs and genomic clones encoding ATG-1622 polypeptide and to isolate cDNA and genomic clones of other genes that have a high sequence similarity to the ATG-1622 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding ATG-1622 polypeptide comprises the steps of screening an appropriate library under stingent hybridization conditions with a labeled probe having the SEQ ID NO: 1 or a fragment thereof (including that of SEQ ID NO: 3), and isolating full-length cDNA and genomic clones containing said polynucleotide sequence. Such hybridization techniques are well known to those of skill in the art. Thus in another aspect, ATG-1622 polynucleotides of the present invention further include a nucleotide sequence comprising a nucleotide sequence that hybridize under stringent condition to a nucleotide sequence having SEQ ID NO: 1 or a fragment thereof (including that of SEQ ID NO:3). Also included with ATG-1622 polypeptides are polypeptide comprising amino acid sequence encoded by nucleotide sequence obtained by the above hybridization condition. Stringent hybridization conditions are as defined above or alternatively conditions under ovemight incubation at 42°C in a solution comprising: 50% formamide, 5xSSC (150mM NaCl, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

**Vectors, Host Cells, Expression**

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *BASIC METHODS IN MOLECULAR BIOLOGY* (1986) and Sambrook et al., *MOLECULAR CLONING: A LABORATORY MANUAL,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as streptococci, staphylococci, *E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL (supra).*

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the ATG-1622 polypeptide is to be expressed for use in screening assays, generally, it is preferred that the polypeptide be produced at the surface of the cell. In this event, the cells may be harvested prior to use in the screening assay. If ATG-1622 polypeptide is secreted into the medium, the medium can be recovered in order to recover and purify the polypeptide; if produced intracellularly, the cells must first be lysed before the polypeptide is recovered. ATG-1622 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

**Diagnostic Assays**

This invention also relates to the use of ATG-1622 polynucleotides for use as diagnostic reagents. Detection of a mutated form of ATG-1622 gene associated with a dysfunction will provide a diagnostic tool that can add to or define a diagnosis of a disease or susceptibility to a disease which results from under-expression, over-expression or altered expression of ATG-1622. Individuals carrying mutations in the ATG-1622 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled ATG-1622 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising ATG-1622 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee *et al., Science,* Vol 274, pp 610-613 (1996)).

The diagnostic assays offer a process for diagnosing or determining a susceptibility to heart disease, hypertension, kidney diseases, obesity, insulin resistance, lipodystrophy, diabetes, and CNS diseases through detection of mutation in the ATG-1622 gene by the methods described.

In addition, heart disease, hypertension, kidney diseases, obesity, insulin resistance, lipodystrophy, diabetes, and CNS diseases, can be diagnosed by methods comprising determining from a sample derived from a subject an abnormally decreased or increased level of ATG-1622 polypeptide or ATG-1622 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an ATG-1622 polypeptide, in a sample derived from a host are well-known to those of skill in the art. Such assay methods include radioimmunoassays, competitive-binding assays, Western Blot analysis and ELISA assays.

**Chromosome Assays**

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes). The differences in the cDNA or genomic sequence between affected and unaffected individuals can also be determined. If a mutation is observed in some or all of the affected individuals but not in any normal individuals, then the mutation is likely to be the causative agent of the disease.

**Antibodies**

The polypeptides of the invention or their fragments or analogs thereof, or cells expressing them can also be used as immunogens to produce antibodies immunospecific for the ATG-1622 polypeptides. The term "immunospecific" means that the antibodies have substantiall greater affinity for the polypeptides of the invention than their affinity for other related polypeptides in the prior art.

Antibodies generated against the ATG-1622 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against ATG-1622 polypeptides may also be employed to treat heart disease, hypertension, kidney diseases, obesity, insulin resistance, lipodystrophy, diabetes, and CNS diseases, among others.

**Vaccines**

Another aspect of the invention relates to a method for inducing an immunological response in a mammal which comprises inoculating the mammal with ATG-1622 polypeptide, or a fragment thereof, adequate to produce antibody and/or T cell immune response to protect said animal from heart disease, hypertension, kidney diseases, obesity, insulin resistance, lipodystrophy, diabetes, and CNS diseases, among others. Yet another aspect of the invention relates to a method of inducing immunological response in a mammal which comprises, delivering ATG-1622 polypeptide via a vector directing expression of ATG-1622 polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a ATG-1622 polypeptide wherein the composition comprises a ATG-1622 polypeptide or ATG-1622 gene. The vaccine formulation may further comprise a suitable carrier. Since ATG-1622 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

**Screening Assays**

The ATG-1622 polypeptide of the present invention may be employed in a screening process for compounds which activate (agonists) or inhibit activation of (antagonists, or otherwise called inhibitors) the ATG-1622 polypeptide of the present invention. Thus, polypeptides of the invention may also be used to assess identify agonist or antagonists from, for example, cells, cell-free preparations, chemical libraries, and natural product mixtures. These agonists or antagonists may be natural substrates, ligands, receptors, etc., as the case may be, of the polypeptide of the present invention; or may be structural or functional mimetics of the polypeptide of the present invention. See Coligan *et al., Current Protocols in Immunology* 1(2):Chapter 5 (1991).

ATG-1622 polypeptides are responsible for many biological functions, including many pathologies. Accordingly, it is desirous to find compounds and drugs which stimulate ATG-1622 polypeptide on the one hand and which can inhibit the function of ATG-1622 polypeptide on the other hand. In general, agonists are employed for therapeutic and prophylactic purposes for such conditions as heart disease, hypertension, kidney diseases, obesity, insulin resistance, lipodystrophy, diabetes, and CNS diseases. Antagonists may be employed for a variety of therapeutic and prophylactic purposes for such conditions as heart disease, hypertension, kidney diseases, obesity, insulin resistance, lipodystrophy, diabetes, and CNS diseases.

In general, such screening procedures may involve using appropriate cells which express the ATG-1622 polypeptide or respond to ATG-1622 polypeptide of the present invention. Such cells include cells from mammals, yeast, *Drosophila* or E. *coli.* Cells which express the ATG-1622 polypeptide (or cell membrane containing the expressed polypeptide) or respond to ATG-1622 polypeptide are then contacted with a test compound to observe binding, or stimulation or inhibition of a functional response. The ability of the cells which were contacted with the candidate compounds is compared with the same cells which were not contacted for ATG-1622 activity.

This full length protein can be used for screening any protein receptors that may interact with it. For example, a GST fusion protein can be produced in bacteria or in a baculovirus system and used for identifying additional receptors that may bind to ATG-1622 protein. In addition, full length ATG-1622 protein can be produced in large amounts in bacterial and/or baculovirus, and protein can be iodinated to look for interacting receptors in a classical binding assay. In addition, this protein is also useful in characterizing potential inhibitors that may inhibit the binding of this protein to its 7-TM receptor in a classical competition experiment.

The assays may simply test binding of a candidate compound wherein adherence to the cells bearing the ATG-1622 polypeptide is detected by means of a label directly or indirectly associated with the candidate compound or in an assay involving competition with a labeled competitor. Further, these assays may test whether the candidate compound results in a signal generated by activation of the ATG-1622 polypeptide, using detection systems appropriate to the cells bearing the ATG-1622 polypeptide. Inhibitors of activation are generally assayed in the presence of a known agonist and the effect on activation by the agonist by the presence of the candidate compound is observed.

The ATG-1622 cDNA, protein and antibodies to the protein may also be used to configure assays for detecting the effect of added compounds on the production of ATG-1622 mRNA and protein in cells. For example, an ELISA may be constructed for measuring secreted or cell associated levels of ATG-1622 protein using monoclonal and polyclonal antibodies by standard methods known in the art, and this can be used to discover agents which may inhibit or enhance the production of ATG-1622 (also called antagonist or agonist, respectively) from suitably manipulated cells or tissues.

The ATG-1622 protein may be used to identify membrane bound or soluble receptors, if any, through standard receptor binding techniques known in the art. These include, but are not limited to, ligand binding and crosslinking assays in which the ATG-1622 is labeled with radioactive isotope (eg 125I), chemically modified (eg biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. In addition to being used for purification and cloning of the receptor, these binding assays can be used to identify agonists and antagonists of ATG-1622 which compete with the binding of ATG-1622 to its receptors. Standard methods for conducting screening assays are well understood in the art.

Examples of potential ATG-1622 polypeptide antagonists include antibodies or, in some cases, oligonucleotides or proteins which are closely related to the ligands, substrates, receptors, etc., as the case may be, of the ATG-1622 polypeptide, e.g., a fragment of the ligands, substrates, receptors, or small molecules which bind to the polypeptide of the present invention but do not elicit a response, so that the activity of the polypeptide is prevented.

**Prophylactic and Therapeutic Methods**

This invention provides methods of treating abnormal conditions such as, heart disease, hypertension, kidney diseases, obesity, insulin resistance, lipodystrophy, diabetes, and CNS diseases, related to both an excess of and insufficient amounts of ATG-1622 polypeptide activity.

If the activity of ATG-1622 polypeptide is in excess, several approaches are available. One approach comprises administering to a subject an inhibitor compound (antagonist) as hereinabove described along with a pharmaceutically acceptable carrier in an amount effective to inhibit the function of the ATG-1622 polypeptide, such as, for example, by blocking the binding of ligands, substrates, etc., or by inhibiting a second signal, and thereby alleviating the abnormal condition. In another approach, soluble forms of ATG-1622 polypeptides still capable of binding the ligand, substrate, etc. in competition with endogenous ATG-1622 polypeptide may be administered. Typical embodiments of such competitors comprise fragments of the ATG-1622 polypeptide.

In another approach, soluble forms of ATG-1622 polypeptides still capable of binding the ligand in competition with endogenous ATG-1622 polypeptide may be administered. Typical embodiments of such competitors comprise fragments of the ATG-1622 polypeptide.

In still another approach, expression of the gene encoding endogenous ATG-1622 polypeptide can be inhibited using expression blocking techniques. Known such techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo*.

For treating abnormal conditions related to an under-expression of ATG-1622 and its activity, several approaches are also available. One approach comprises administering to a subject a therapeutically effective amount of a compound which activates ATG-1622 polypeptide, i.e., an agonist as described above, in combination with a pharmaceutically acceptable carrier, to thereby alleviate the abnormal condition. Alternatively, gene therapy may be employed to effect the endogenous production of ATG-1622 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer a therapeutic amount of ATG-1622 polypeptides in combination with a suitable pharmaceutical carrier.

**Formulation and Administration**

Peptides, such as the soluble form of ATG-1622 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide ex *vivo,* and for example, by the use of a retroviral plasmid vector. The cells are then introduced into the subject.

**Examples:**

The examples below are carried out using standard techniques, which are well known and routine to those of skill in the art, except where otherwise described in detail. The examples illustrate, but do not limit the invention.

***Example 1: Expression and Purification of ATG-1622 in E. coli***

ATG-1622 encodes a secreted human protein. It has a secreted signal peptide at the N-terminal end that will be cleaved. Thus, a prokaryotic expression system (E.Coli) will be used to generate large quantities of pure ATG-1622 protein with the C-terminal of the bacterial expressed ATG-1622 being tagged with a histidine tag. The detailed experimental procedure is described below.

The bacterial expression vector pQE60 is used for bacterial expression in this example. (QIAGEN, Inc., 9259 Eton Avenue, Chatsworth, CA, 91311). pQE60 encodes ampicillin antibiotic resistance ("Amp$^r$") and contains a bacterial origin of replication ("on"), an IPTG inducible promoter, a ribosome binding site ("RBS"), six codons encoding histidine residues that allow affinity purification using nickel-nitrilo-tri-acetic acid ("Ni-NTA") affinity resin sold by QIAGEN, Inc., *supra,* and suitable single restriction enzyme cleavage sites. These elements are arranged such that an inserted DNA fragment encoding a polypeptide expresses that polypeptide with the six His residues (i.e., a "6 X His tag") covalently linked to the carboxyl terminus of that polypeptide.

The DNA sequence encoding the desired portion **ATG-1622** protein lacking the hydrophobic leader sequence is amplified from the cDNA clone using PCR oligonucleotide primers which anneal to the amino terminal sequences of the desired portion of the **ATG-1622** protein and to sequences in the construct 3' to the cDNA coding sequence. Additional nucleotides containing restriction sites to facilitate cloning in the pQE60 vector are added to the 5' and 3' sequences, respectively.

For cloning the mature protein, the 5' primer has the sequence 5' **GAATTCATGCTGCAGGGCCCTGGCTCT** 3' (SEQ ID NO:5) containing the underlined **EcoR I** restriction site followed by **21** nucleotides complementary to the amino terminal coding sequence of the mature **ATG-1622** sequence in Figure 1. One of ordinary skill in the art would appreciate, of course, that the point in the protein coding sequence where the 5' primer begins may be varied to amplify a DNA segment encoding any desired portion of the complete protein shorter or longer than the mature form. The 3' primer has the sequence 5' **GAATTCGCACTGCAGCTTGCGGATGCT** 3' (SEQ ID NO: **6**) containing the underlined **EcoR I** restriction site followed by **21** nucleotides complementary to the 3' end of the coding sequence immediately before the stop codon in the **ATG-1622** DNA sequence in Figure 1, with the coding sequence aligned with the restriction site so as to maintain its reading frame with that of the six His codons in the pQE60 vector.

The amplified **ATG-1622** DNA fragment and the vector pQE60 are digested with **EcoR I** and the digested DNAs are then ligated together. Insertion of the **ATG-1622** DNA into the restricted pQE60 vector places the **ATG-1622** protein coding region downstream from the IPTG-inducible promoter and in-frame with an initiating AUG and the six histidine codons.

### *Example 2: Expression and Purification of untagged ATG-1622 in E. coli*

In case that the tag may interfere with biological activity, we will also express ATG-1622 without tag at either end in E.Coli. The following is the detailed procedure.

The bacterial expression vector pQE60 is used for bacterial expression in this example. (QIAGEN, Inc., 9259 Eton Avenue, Chatsworth, CA, 91311). pQE60 encodes ampicillin antibiotic resistance ("Amp$^r$") and contains a bacterial origin of replication ("ori"), an IPTG inducible promoter, a ribosome binding site ("RBS"), six codons encoding histidine residues that allow affinity purification using nickel-nitrilo-tri-acetic acid ("Ni-NTA") affinity resin sold by QIAGEN, Inc., *supra,* and suitable single restriction enzyme cleavage sites. These elements are arranged such that a DNA fragment encoding a polypeptide may be inserted in such as way as to produce that polypeptide with the six His residues (i.e., a "6 X His tag") covalently linked to the carboxyl terminus of that polypeptide. However, in this example, the polypeptide coding sequence is inserted such that translation of the six His codons is prevented and, therefore, the polypeptide is produced with no 6 X His tag.

The DNA sequence encoding the desired portion of the **ATG-1622** protein lacking the hydrophobic leader sequence is amplified from the cDNA clone using PCR oligonucleotide primers which anneal to the amino terminal sequences of the desired portion of the **ATG-1622** protein and to sequences in the construct 3' to the cDNA coding sequence. Additional nucleotides containing restriction sites to facilitate cloning in the pQE60 vector are added to the 5' and 3' sequences, respectively.

For cloning the mature protein, the 5' primer has the sequence 5' **GAATTCATGCTGCAGGGCCCTGGCTCT** 3' (SEQ ID NO:**5**) containing the underlined **EcoR I** restriction site followed by **21** nucleotides complementary to the amino terminal coding sequence of the mature **ATG-1622** sequence in Figure 1. One of ordinary skill in the art would appreciate, of course, that the point in the protein coding sequence where the 5' primer begins may be varied to amplify a desired portion of the complete protein shorter or longer than the mature form. The 3' primer has the sequence 5' **GAATTCCTAGCACTGCAGCTTGCGGAT** 3' (SEQ ID NO: **7**) containing the underlined **EcoRI** restriction site followed by **21** nucleotides complementary to the 3' end of the coding sequence immediately before the stop codon in the **ATG-1622** DNA sequence in Figure 1.

The amplified **ATG-1622** DNA fragments and the vector pQE60 are digested with **EcoRI** and the digested DNAs are then ligated together. Insertion of the **ATG-1622** DNA into the restricted pQE60 vector places the **ATG-1622** protein coding region including its associated stop codon downstream from the IPTG-inducible promoter and in-frame with an initiating AUG. The associated stop codon prevents translation of the six histidine codons downstream of the insertion point.

The ligation mixture is transformed into competent *E. coli* cells using standard procedures such as those described in Sambrook *et al., Molecular Cloning: a Laboratory Manual, 2nd Ed.;* Cold Spring Harbor Laboratory Press, Cold Spring Harbor, NY (1989). *E. coli* strain M15/rep4, containing multiple copies of the plasmid pREP4, which expresses the lac repressor and confers kanamycin resistance ("Kan$^r$"), is used in carrying out the illustrative example described herein. This strain, which is only one of many that are suitable for expressing **ATG-1622** protein, is available commercially from QIAGEN, Inc., *supra.* Transformants are identified by their ability to grow on LB plates in the presence of ampicillin and kanamycin. Plasmid DNA is isolated from resistant colonies and the identity of the cloned DNA confirmed by restriction analysis, PCR and DNA sequencing.

Clones containing the desired constructs are grown overnight ("O/N") in liquid culture in LB media supplemented with both ampicillin (100 mg/ml) and kanamycin (25 mg/ml). The O/N culture is used to inoculate a large culture, at a dilution of approximately 1:25 to 1:250. The cells are grown to an optical density at 600 nm ("OD600") of between 0.4 and 0.6. Isopropyl-b-D-thiogalactopyranoside ("IPTG") is then added to a final concentration of 1 mM to induce transcription from the lac repressor sensitive promoter, by inactivating the lacI repressor. Cells subsequently are incubated further for 3 to 4 hours. Cells then are harvested by centrifugation.

The cells are then stirred for 3-4 hours at 4°C in 6M guanidine-HCl, pH8. The cell debris is removed by centrifugation, and the supernatant containing the **ATG-1622** is dialyzed against 50 mM Na-acetate buffer pH6, supplemented with 200 mM NaCl. Alternatively, the protein can be successfully refolded by dialyzing it against 500 mM NaCl, 20% glycerol, 25 mM Tris/HCl pH7.4, containing protease inhibitors. After renaturation the protein can be purified by ion exchange, hydrophobic interaction and size exclusion chromatography. Alternatively, an affinity chromatography step such as an antibody column can be used to obtain pure **ATG-1622** protein. The purified protein is stored at 4°C or frozen at -80°C.

***Example 3: Cloning and Expression of ATG-1622 protein in a Baculovirus Expression System***

In this illustrative example, the plasmid shuttle vector pA2 is used to insert the cloned DNA encoding the complete protein, including its naturally associated secretory signal (leader) sequence, into a baculovirus to express the mature **ATG-1622** protein, using standard methods as described in Summers *et al., A Manual of Methods for Baculovirus Vectors and Insect Cell Culture Procedures,* Texas Agricultural Experimental Station Bulletin No. 1555 (1987). This expression vector contains the strong polyhedrin promoter of the *Autographa californica* nuclear polyhedrosis virus (AcMNPV) followed by convenient restriction sites such as BamHI and Asp718. The polyadenylation site of the simian virus 40 ("SV40") is used for efficient polyadenylation. For easy selection of recombinant virus, the plasmid contains the beta-galactosidase gene from *E. coli* under control of a weak Drosophila promoter in the same orientation, followed by the polyadenylation signal of the polyhedrin gene. The inserted genes are flanked on both sides by viral sequences for cell-mediated homologous recombination with wild-type viral DNA to generate viable virus that express the cloned polynucleotide.

Many other baculovirus vectors could be used in place of the vector above, such as pAc373, pVL941 and pAcIM1, as one skilled in the art would readily appreciate, as long as the construct provides appropriately located signals for transcription, translation, secretion and the like, including a signal peptide and an in-frame AUG as required. Such vectors are described, for instance, in Luckow *et al., Virology 170*:31-39.

The cDNA sequence encoding the full length **ATG-1622** protein in the clone, including the AUG initiation codon and the naturally associated leader sequence shown in Table 1 (SEQ ID NO:1), is amplified using PCR oligonucleotide primers corresponding to the 5' and 3' sequences of the gene. The 5' primer has the sequence 5' **GAATTCCAAATGCTGCAGGGCCCTGGCTCT** 3' (SEQ ID NO:**8**) containing the underlined **EcoRI** restriction enzyme site, an efficient signal for initiation of translation in eukaryotic cells, as described by Kozak, M., *J. Mol. Biol. 196*:947-950 (1987), followed by **21** bases of the sequence of the complete **ATG-1622** protein shown in Figure 1, beginning with the AUG initiation codon. The 3' primer has the sequence 5' **GAATTCCTACCACTGCAGCT'TGCGGAT** 3' (SEQ ID NO:**7**) containing the underlined **EcoRI** restriction site followed by **21** nucleotides complementary to the 3' end of the coding sequence immediately before the stop codon in the **ATG-1622** DNA sequence in Figure 1.

The amplified fragment is isolated from a 1% agarose gel using a commercially available kit ("Geneclean," BIO 101 Inc., La Jolla, Ca.). The fragment then is digested with **EcoRI** and again is purified on a 1% agarose gel. This fragment is designated herein "F1".

The plasmid is digested with the restriction enzymes **EcoRI** and optionally, can be dephosphorylated using calf intestinal phosphatase, using routine procedures known in the art. The DNA is then isolated from a 1% agarose gel using a commercially available kit ("Geneclean" BIO 101 Inc., La Jolla, Ca.). This vector DNA is designated herein "V1".

Fragment F1 and the dephosphorylated plasmid V1 are ligated together with T4 DNA ligase. *E. coli* HB101 or other suitable *E. coli* hosts such as XL-1 Blue (Stratagene Cloning Systems, La Jolla, CA) cells are transformed with the ligation mixture and spread on culture plates. Bacteria are identified that contain the plasmid with the human **ATG-1622** gene using the PCR method, in which one of the primers that is used to amplify the gene and the second primer is from well within the vector so that only those bacterial colonies containing the **ATG-1622** gene fragment will show amplification of the DNA. The sequence of the cloned fragment is confirmed by DNA sequencing. This plasmid is designated herein pBac**ATG-1622**.

5 mg of the plasmid pBac**ATG-1622** is co-transfected with 1.0 mg of a commercially available linearized baculovirus DNA ("BaculoGoldÔ baculovirus DNA", Pharmingen, San Diego, CA.), using the lipofection method described by Felgner *et al., Proc. Natl. Acad. Sci. USA 84*:7413-7417 (1987). 1 mg of BaculoGoldÔ virus DNA and 5 mg of the plasmid pBacATG-1622 are mixed in a sterile well of a microtiter plate containing 50 ml of serum-free Grace's medium (Life Technologies Inc., Gaithersburg, MD). Afterwards, 10 ml Lipofectin plus 90 ml Grace's medium are added, mixed and incubated for 15 minutes at room temperature. Then the transfection mixture is added drop-wise to Sf9 insect cells (ATCC CRL 1711) seeded in a 35 mm tissue culture plate with 1 ml Grace's medium without serum. The plate is rocked back and forth to mix the newly added solution. The plate is then incubated for 5 hours at 27°C. After 5 hours the transfection solution is removed from the plate and 1 ml of Grace's insect medium supplemented with 10% fetal calf serum is added. The plate is put back into an incubator and cultivation is continued at 27°C for four days.

After four days the supernatant is collected and a plaque assay is performed, as described by Summers and Smith, *supra.* An agarose gel with "Blue Gal" (Life Technologies Inc., Gaithersburg) is used to allow easy identification and isolation of gal-expressing clones, which produce blue-stained plaques. (A detailed description of a "plaque assay" of this type can also be found in the user's guide for insect cell culture and baculovirology distributed by Life Technologies Inc., Gaithersburg, page 9-10). After appropriate incubation, blue stained plaques are picked with the tip of a micropipettor (e.g., Eppendorf). The agar containing the recombinant viruses is then resuspended in a microcentrifuge tube containing 200 ml of Grace's medium and the suspension containing the recombinant baculovirus is used to infect Sf9 cells seeded in 35 mm dishes. Four days later the supernatants of these culture dishes are harvested and then they are stored at 4°C. The recombinant virus is called V-ATG-1622

To verify the expression of the **ATG-1622** gene, Sf9 cells are grown in Grace's medium supplemented with 10% heat inactivated FBS. The cells are infected with the recombinant baculovirus V-**ATG-1622** at a multiplicity of infection ("MOI") of about 2. Six hours later the medium is removed and is replaced with SF900 II medium minus methionine and cysteine (available from Life Technologies Inc., Rockville, MD). If radiolabeled proteins are desired, 42 hours later, 5 mCi of$^{35}$S-methionine and 5 mCi $^{35}$S-cysteine (available from Amersham) are added. The cells are further incubated for 16 hours and then they are harvested by centrifugation. The proteins in the supernatant as well as the intracellular proteins are analyzed by SDS-PAGE followed by autoradiography (if radiolabeled). Microsequencing of the amino acid sequence of the amino terminus of purified protein may be used to determine the amino terminal sequence of the mature protein and thus the cleavage point and length of the secretory signal peptide.

### *Example 4: Cloning and Expression of ATG-1622 in Mammalian Cells*

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as PSVL and PMSG (Pharmacia, Uppsala, Sweden), pRSVcat (ATCC 37152), pSV2dhfr (ATCC 37146) and pBC12MI (ATCC 67109). Mammalian host cells that could be used include, human Hela 293, H9 and Jurkat cells, mouse NIH3T3 and C127 cells, Cos 1, Cos 7 and CV 1, quail QC1-3 cells, mouse L cells and Chinese hamster ovary (CHO) cells.

Alternatively, the gene can be expressed in stable cell lines that contain the gene integrated into a chromosome. The co-transfection with a selectable marker such as dhfr, gpt, neomycin, or hygromycin allows the identification and isolation of the transfected cells.

The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy *et al., Biochem J. 227*:277-279 (1991); Bebbington *et al., Bio/Technology* 10:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

The expression vectors pC1 and pC4 contain the strong promoter (LTR) of the Rous Sarcoma Virus (Cullen *et al., Molecular and Cellular Biology,* 438447 (March, 1985)) plus a fragment of the CMV-enhancer (Boshart *et al., Cell 41*: 521-530 (1985)). Multiple cloning sites, e.g., with the restriction enzyme cleavage sites BamHI, XbaI and Asp718, facilitate the cloning of the gene of interest. The vectors contain in addition the 3' intron, the polyadenylation and termination signal of the rat preproinsulin gene.

***Example 4(a): Cloning and Expression in COS Cells***

The expression plasmid, **pATG-1622** HA, is made by cloning a cDNA encoding **ATG-1622** into the expression vector pcDNAI/Amp or pcDNAIII (which can be obtained from Invitrogen, Inc.).

The expression vector pcDNAI/amp contains: (1) an *E. coli* origin of replication effective for propagation in *E. coli* and other prokaryotic cells; (2) an ampicillin resistance gene for selection of plasmid-containing prokaryotic cells; (3) an SV40 origin of replication for propagation in eukaryotic cells; (4) a CMV promoter, a polylinker, an SV40 intron; (5) several codons encoding a hemagglutinin fragment (i.e., an "HA" tag to facilitate purification) followed by a termination codon and polyadenylation signal arranged so that a cDNA can be conveniently placed under expression control of the CMV promoter and operably linked to the SV40 intron and the polyadenylation signal by means of restriction sites in the polylinker. The HA tag corresponds to an epitope derived from the influenza hemagglutinin protein described by Wilson *et al., Cell 37:*767 (1984). The fusion of the HA tag to the target protein allows easy detection and recovery of the recombinant protein with an antibody that recognizes the HA epitope. pcDNAIII contains, in addition, the selectable neomycin marker.

A DNA fragment encoding the **ATG-1622** is cloned into the polylinker region of the vector so that recombinant protein expression is directed by the CMV promoter. The plasmid construction strategy is as follows. The **ATG-1622** cDNA of the clone is amplified using primers that contain convenient restriction sites, much as described above for construction of vectors for expression of **ATG-1622** in *E. coli.* Suitable primers include the following, which are used in this example. The 5' primer, containing the underlined **EcoRI** site, a Kozak sequence, an AUG start codon and **6** codons of the 5' coding region of the complete **ATG-1622** has the following sequence: 5' **<u>GAATTC</u>CAAATGCTGCAG-GGCCCTGGCTCT** 3' (SEQ ID NO: **8**). The 3' primer, containing the underlined **EcoRI** site, a stop codon, and **21** bp of 3' coding sequence has the following sequence (at the 3' end): 5' **<u>GAATTC</u>CTAGCACTGCAGCTTGCGGAT** 3' (SEQ ID NO: 7).

The PCR amplified DNA fragment and the vector, pcDNAI/Amp, are digested with **EcoRI** and then ligated. The ligation mixture is transformed into *E. coli* strain SURE (available from Stratagene Cloning Systems, 11099 North Torrey Pines Road, La Jolla, CA 92037), and the transformed culture is plated on ampicillin media plates which then are incubated to allow growth of ampicillin resistant colonies. Plasmid DNA is isolated from resistant colonies and examined by restriction analysis or other means for the presence of the **ATG1622**-encoding fragment.

For expression of recombinant **ATG-1622** COS cells are transfected with an expression vector, as described above, using DEAE-DEXTRAN, as described, for instance, in Sambrook *et al., Molecular Cloning: a Laboratory Manual,* Cold Spring Laboratory Press, Cold Spring Harbor, New York (1989). Cells are incubated under conditions for expression of **ATG-1622** by the vector.

Expression of the **ATG-1622**-HA fusion protein is detected by radiolabeling and immunoprecipitation, using methods described in, for example Harlow *et al., Antibodies: A Laboratory Manual, 2nd Ed.;* Cold Spring Harbor Laboratory Press, Cold Spring Harbor, New York (1988). To this end, two days after transfection, the cells are labeled by incubation in media containing $^{35}$S-cysteine for 8 hours. The cells and the media are collected, and the cells are washed and lysed with detergent-containing RIPA buffer: 150 mM NaCl, 1% NP40, 0.1% SDS, 0.5% DOC, 50 mM TRIS, pH 7.5, as described by Wilson *et al.* cited above. Proteins are precipitated from the cell lysate and from the culture media using an HA-specific monoclonal antibody. The precipitated proteins then are analyzed by SDS-PAGE and autoradiography. An expression product of the expected size is seen in the cell lysate, which is not seen in negative controls.

***Example 4(b): Cloning and Expression in CHO Cells***

The vector pC4 is used for the expression of **ATG-1622** protein. Plasmid pC4 is a derivative of the plasmid pSV2-dhfr (ATCC Accession No. 37146). The plasmid contains the mouse DHFR gene under control of the SV40 early promoter. Chinese hamster ovary- or other cells lacking dihydrofolate activity that are transfected with these plasmids can be selected by growing the cells in a selective medium (alpha minus MEM, Life Technologies) supplemented with the chemotherapeutic agent methotrexate. The amplification of the DHFR genes in cells resistant to methotrexate (MTX) has been well documented (see, e.g., Alt, F. W., Kellems, R M., Bertino, J. R., and Schimke, R. T., 1978, *J Biol. Chem.* 253:1357-1370, Hamlin, J. L. and Ma, C. 1990, *Biochem. et Biophys. Acta, 1097*:107-143, Page, M. J. and Sydenham, M.A. 1991, *Biotechnology 9*:64-68). Cells grown in increasing concentrations of MIX develop resistance to the drug by overproducing the target enzyme, DHFR, as a result of amplification of the DHFR gene. If a second gene is linked to the DHFR gene, it is usually co-amplified and over-expressed. It is known in the art that this approach may be used to develop cell lines carrying more than 1,000 copies of the amplified gene(s). Subsequently, when the methotrexate is withdrawn, cell lines are obtained which contain the amplified gene integrated into one or more chromosome(s) of the host cell.

Plasmid pC4 contains for expressing the gene of interest the strong promoter of the long terminal repeat (LTR) of the Rous Sarcoma Virus (Cullen, *et al., Molecular and Cellular Biology,* March 1985:438-447) plus a fragment isolated from the enhancer of the immediate early gene of human cytomegalovirus (CMV) (Boshart *et al., Cell 41*:521-530 (1985)). Downstream of the promoter are BamHI, XbaI, and Asp718 restriction enzyme cleavage sites that allow integration of the genes. Behind these cloning sites the plasmid contains the 3' intron and polyadenylation site of the rat preproinsulin gene. Other high efficiency promoters can also be used for the expression, e.g., the human b-actin promoter, the SV40 early or late promoters or the long terminal repeats from other retroviruses, e.g., HIV and HTLVI. Clontech's Tet-Off and Tet-On gene expression systems and similar systems can be used to express the **ATG-1622** in a regulated way in mammalian cells (Gossen, M., & Bujard, H. 1992, *Proc. Natl. Acad. Sci. USA 89*: 5547-5551). For the polyadenylation of the mRNA other signals, e.g., from the human growth hormone or globin genes can be used as well. Stable cell lines carrying a gene of interest integrated into the chromosomes can also be selected upon co-transfection with a selectable marker such as gpt, G418 or hygromycin. It is advantageous to use more than one selectable marker in the beginning, e.g., G418 plus methotrexate.

The plasmid pC4 is digested with the restriction enzymes **EcoRI** and then dephosphorylated using calf intestinal phosphatase by procedures known in the art. The vector is then isolated from a 1% agarose gel.

The DNA sequence encoding the complete **ATG-1622** protein including its leader sequence is amplified using PCR oligonucleotide primers corresponding to the 5' and 3' sequences of the gene. The 5' primer has the sequence 5' **GAATTCCAAATGCTGCAGGGCCCTGGCTCT** 3' (SEQ ID NO: **8**) containing the underlined **EcoRI** restriction enzyme site followed by an efficient signal for initiation of translation in eukaryotes, as described by Kozak, M., *J. Mol. Biol. 196*:947-950 (1987), and **21** bases of the coding sequence of **ATG-1622** shown in Table 1 (SEQ ID NO:1). The 3' primer has the sequence 5' **GAATTCCTAGCACTGCAGCTTGCGGAT** 3' (SEQ ID NO: **7**) containing the underlined **EcoRI** restriction site followed 21 nucleotides complementary to the 3' region of the **ATG-1622** gene shown in Table 1 (SEQ ID NO:1).

The amplified fragment is digested with the endonucleases **EcoRI** and then purified again on a 1% agarose gel. The isolated fragment and the dephosphorylated vector are then ligated with T4 DNA ligase. *E. coli* HB101 or XL-1 Blue cells are then transformed and bacteria are identified that contain the fragment inserted into plasmid pC4 using, for instance, restriction enzyme analysis.

Chinese hamster ovary cells lacking an active DHFR gene are used for transfection. 5 mg of the expression plasmid pC4 is cotransfected with 0.5 mg of the plasmid pSV2-neo using lipofectin (Felgner *et al., supra).* The plasmid pSV2neo contains a dominant selectable marker, the neo gene from Tn5 encoding an enzyme that confers resistance to a group of antibiotics including G418. The cells are seeded in alpha minus MEM supplemented with 1 mg/ml G418. After 2 days, the cells are trypsinized and seeded in hybridoma cloning plates (Greiner, Germany) in alpha minus MEM supplemented with 10, 25, or 50 ng/ml of metohrexate plus 1 mg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 mM, 2 mM, 5 mM, 10 mM, 20 mM). The same procedure is repeated until clones are obtained which grow at a concentration of 100 - 200 mM. Expression of the desired gene product is analyzed, for instance, by SDS-PAGE and Western blot or by reverse phase HPLC analysis.

***Example 5: Tissue distribution of ATG-1622 mRNA pression***

Northern blot analysis is carried out to examine **ATG-1622** gene expression in human tissues, using methods described by, among others, Sambrook *et al.,* cited above. A cDNA probe containing the entire nucleotide sequence of the **ATG-1622** protein (SEQ ID NO: 1) is labeled with $^{32}P$ using the *redi*primeÔ DNA labeling system (Amersham Life Science), according to manufacturer's instructions. After labeling, the probe is purified using a CHROMA SPIN-100Ô column (Clontech Laboratories, Inc.), according to manufacturer's protocol number PT1200-1. The purified labeled probe is then used to examine various human tissues for **ATG-1622** mRNA.

Multiple Tissue Northern (MTN) blots containing various human tissues (H) or human immune system tissues (IM) are obtained from Clontech and are examined with the labeled probe using ExpressHybÔ hybridization solution (Clontech) according to manufacturer's protocol number PT1190-1. Following hybridization and washing, the blots are mounted and exposed to film at -70°C overnight, and films developed according to standard procedures.

***Example 6***

From Northern mRNA expression studies, we have now found that in both rodent (mice and rat) and human, ATG-1622 is highly expressed in adipose tissue. This expression is concentrated in the undifferentiated preadipocytes fraction, while little expression is observed in mature adipocytes. This finding implies that the adipocyte differentiation is accompanied by the cocomittant decrease of ATG-1622 expression. Thus, approaches that enhance the expression of ATG-1622, or administration of ATG-1622, might be used to inhibit adipocyte differentiation process, thus ATG-1622 might be useful in treating or preventing the onset of obesity.

In addition, we also observed that in obese mice fat, the expression of ATG-1622 mRNA is significantly reduced. This is consistent with our observation that mature adipocyte does not contain ATG-1622. Thus, elevation of ATG-1622 may be benificial for obesity syndrom.

## Claims

1. An isolated polynucleotide comprising a nucleotide sequence that has at least 80% identity to a nucleotide sequence encoding the ATG-1622 polypeptide of SEQ ID NO:2 over its entire length; or a nucleotide sequence complementary to said isolated polynucleotide.

2. The polynucleotide of claim 1 which is DNA or RNA.

3. The polynucleotide of claim 1 wherein said polynucleotide comprises a nucleotide sequence that has at least 80% identical to that of SEQ ID NO:1 over its entire length.

4. The polynucleotide of claim 3 wherein said nucleotide sequence comprises the ATG-1622 polypeptide encoding sequence contained in SEQ ID NO:1.

5. The polynucleotide of claim 3 which is polynucleotide of SEQ ID NO: 1.

6. A DNA or RNA molecule comprising an expression system, wherein said expression system is capable of producing a ATG-1622 polypeptide comprising an amino acid sequence, which has at least 80% identity with the polypeptide of SEQ ID NO:2 when said expression system is present in a compatible host cell.

7. A host cell comprising the expression system of claim 6.

8. A process for producing a ATG-1622 polypeptide comprising culturing a host of claim 7 under conditions sufficient for the production of said polypeptide and recovering the polypeptide from the culture.

9. A process for producing a cell which produces a ATG-1622 polypeptide thereof comprising transforming or transfecting a host cell with the expression system of claim 6 such that the host cell, under appropriate culture conditions, produces a ATG-1622 polypeptide.

10. A ATG-1622 polypeptide comprising an amino acid sequence which is at least 80% identical to the amino acid sequence of SEQ ID NO:2 over its entire length.

11. The polypeptide of claim 10 which comprises the amino acid sequence of SEQ ID NO:2.

12. An antibody immunospecific for the ATG-1622 polypeptide of claim 10.

13. A method for the treatment of a subject in need of enhanced activity or expression of ATG-1622 polypeptide of claim 10 comprising:

(a) administering to the subject a therapeutically effective amount of an agonist to said polypeptide; and/or
(b) providing to the subject an isolated polynucleotide comprising a nucleotide sequence that has at least 80% identity to a nucleotide sequence encoding the ATG-1622 polypeptide of SEQ ID NO:2 over its entire length; or a nucleotide sequence complementary to said nucleotide sequence in a form so as to effect production of said polypeptide activity *in vivo.*

14. A method for the treatment of a subject having need to inhibit activity or expression of ATG-1622 polypeptide of claim 10 comprising:

(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate , or receptor.

15. A process for diagnosing a disease or a susceptibility to a disease in a subject related to expression or activity of ATG-1622 polypeptide of claim 10 in a subject comprising:

(a) determining the presence or absence of a mutation in the nucleotide sequence encoding said ATG-1622 polypeptide in the genome of said subject; and/or
(b) analyzing for the presence or amount of the ATG-1622 polypeptide expression in a sample derived from said subject.

16. A method for identifying compounds which inhibit (antagonize) or agonize the ATG-1622 polypeptide of claim 10 which comprises:

(a) contacting a candidate compound with cells which express the ATG-1622 polypeptide (or cell membrane expressing ATG-1622 polypeptide) or respond to ATG-1622 polypeptide; and
(b) observing the binding, or stimulation or inhibition of a functional response; or comparing the ability of the cells (or cell membrane) which were contacted with the candidate compounds with the same cells which were not contacted for ATG-1622 polypeptide activity.

17. An agonist identified by the method of claim 16.

18. An antagonist identified by the method of claim 16.

European Patent Office

## PARTIAL EUROPEAN SEARCH REPORT

which under Rule 45 of the European Patent Convention shall be considered, for the purposes of subsequent proceedings, as the European search report

Application Number EP 98 30 3809

### DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,D | SHIROZU M ET AL: "CHARACTERIZATION OF NOVEL SECRETED AND MEMBRANE PROTEINS ISOLATED BY THE SIGNAL SEQUENCE TRAP METHOD" GENOMICS, vol. 37, no. 3, 1 November 1996, pages 273-280, XP002054773 * the whole document * & "AC D50462" EMBL DATABASE,22 December 1996, HEIDELBERG * the whole document * --- | 1-3, 6-10,12, 17,18 | C12N15/12 C12N15/85 C12N5/10 C12Q1/68 C07K14/47 C07K16/18 A61K38/17 A61K48/00 G01N33/50 |
| A | HOANG B ET AL: "PRIMARY STRUCTURE AND TISSUE DISTRIBUTION OF FRZB, A NOVEL PROTEIN RELATED TO DROSOPHILA FRIZZLED, SUGGEST A ROLE IN SKELETAL MORPHOGENESIS" JOURNAL OF BIOLOGICAL CHEMISTRY, vol. 271, no. 42, 18 October 1996, pages 26131-26137, XP002054777 * the whole document * --- | 1-18 | |
| | -/-- | | TECHNICAL FIELDS SEARCHED (Int.Cl.6) C07K C12N C12Q A61K G01N |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claims 13,14 are directed to a method of treatment of the human/animal body (Article 52(4) EPC), the search has been carried out and based on the alleged effects of the compound/composition.

| Place of search | Date of completion of the search | Examiner |
|---|---|---|
| THE HAGUE | 16 September 1998 | Kania, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 98 30 3809

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A,D | RATTNER A ET AL: "A FAMILY OF SECRETED PROTEINS CONTAINS HOMOLOGY TO THE CYSTEINE-RICH LIGAND-BINDING DOMAIN OF FRIZZLED RECEPTORS" PROCEEDINGS OF THE NATIONAL ACADEMY OF SCIENCES OF USA, vol. 94, no. 7, 1 April 1997, pages 2859-2863, XP002054779 * the whole document * --- | 1-18 | |
| P,X | MELKONYAN H S ET AL: "SARPS: A FAMILY OF SECRETED APOPTOSIS-RELATED PROTEINS" PROCEEDINGS OF THE NATIONAL ACADEMY OF SCIENCES OF USA, vol. 94, no. 25, 9 December 1997, pages 13636-13641, XP002057810 * the whole document * --- | 1-4, 6-10,12 | TECHNICAL FIELDS SEARCHED (Int.Cl.6) |
| P,X | WO 98 13493 A (UMANSKY SAMUIL ;MELKONYAN HOVSEP (RU); LXR BIOTECHNOLOGY INC (US)) 2 April 1998 * see the whole document, esp. SEQ ID 2,3 * --- | 1-18 | |
| E | WO 98 35043 A (GENETICS INST) 13 August 1998 * the whole document * ----- | 1-18 | |

EPO FORM 1503 03.82 (P04C10)